(19) Europäisches Patentamt
European Patent Office
Office européen des brevets

(11) Veröffentlichungsnummer: **0 137 434 B1**

# (12) EUROPÄISCHE PATENTSCHRIFT

(45) Veröffentlichungstag der Patentschrift: **29.01.92**

(51) Int. Cl.⁵: **C07J 1/00**, C07J 53/00, C07J 51/00, C07J 69/00, C07J 63/00, C07J 21/00

(21) Anmeldenummer: **84111685.8**

(22) Anmeldetag: **29.09.84**

Die Akte enthält technische Angaben, die nach dem Eingang der Anmeldung eingereicht wurden und die nicht in dieser Patentschrift enthalten sind.

## (54) Estran- und Androstan-Derivate, Verfahren zu ihrer Herstellung und diese Verbindungen enthaltende Präparate.

(30) Priorität: **10.10.83 DE 3337179**

(43) Veröffentlichungstag der Anmeldung:
**17.04.85 Patentblatt 85/16**

(45) Bekanntmachung des Hinweises auf die Patenterteilung:
**29.01.92 Patentblatt 92/05**

(84) Benannte Vertragsstaaten:
**AT BE CH DE FR GB IT LI LU NL SE**

(56) Entgegenhaltungen:

**THE JOURNAL OF NUCLEAR MEDICINE, Band 23, Nr. 5, Mai 1982, Seiten 431-436, New York, US; R.H. HANSON et al.: "E-17delta[125 ]Iodovinylestradiol: an estrogen-receptor-seeking radiopharmaceutical"**

**J.Clin.Chem. Clin. Brochem.,25,1987,pp107-112;Angew.Chem. 96(1984), pp720-721.**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Hofmeister, Helmut, Dr.**
**Weislingenstrasse 4**
**W-1000 Berlin 28(DE)**
Erfinder: **Laurent, Henry, Dr.**
**Glambecker Weg 21**
**W-1000 Berlin 28(DE)**
Erfinder: **Schulze, Paul Eberhard, Dr.**
**Endestrasse 30**
**W-1000 Berlin 39(DE)**
Erfinder: **Annen, Klaus, Dr.**
**Osthofstrasse 80**
**W-4400 Münster-Albachten(DE)**
Erfinder: **Pollow, Kunhard, Prof. Dr.**
**Lion Feuchtwangerstrasse 63**
**W-6500 Mainz-Hechtsheim(DE)**
Erfinder: **Manz, Bernhard, Dr.**
**August-Bebel-Strasse 26**
**W-6500 Mainz-Bretzenheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Erfinder: **Grill, Hans-Jörg**
**Neue Universitätsstrasse 5**
**W-6500 Mainz(DE)**

## Beschreibung

Die Erfindung betrifft
Estran- und Androstan-Derivate der allgemeinen Formel I

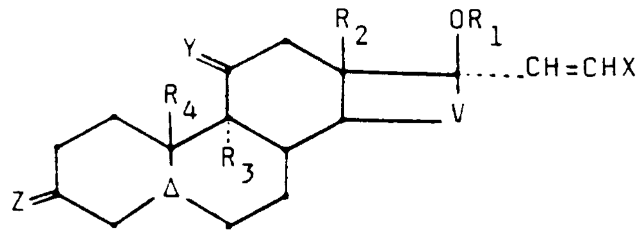

(I),

worin

| | |
|---|---|
| X | ein Bromatom, ein Iodatom, eine Triphenylzinngruppe oder eine Trialkylzinngruppe mit 1 bis 6 Kohlenstoffatomen je Alkylrest, |
| Y | zwei Wasserstoffatome oder eine Methylengruppe, |
| Z | zwei Wasserstoffatome, eine Oxogruppe oder eine Alkylendioxygruppe mit 2 bis 6 Kohlenstoffatomen und |
| V | eine Ethylengruppe, eine Vinylengruppe, eine 1,3-Propylengruppe oder eine Cyclopropylengruppe darstellen, worin |
| $R_1$ | ein Wasserstoffatom oder einen gegebenenfalls durch eine Oxygruppe unterbrochenen oder durch eine Oxogruppe substituierten Kohlenwasserstoffrest mit maximal 8 Kohlenstoffatomen und |
| $R_2$ | eine Methyl- oder Ethylgruppe bedeuten, worin |
| $R_3$ und $R_4$ | eine Kohlenstoff-Kohlenstoff-Bindung darstellt oder worin $R_3$ ein Wasserstoffatom bedeutet und |
| $R_4$ | ein Wasserstoffatom oder eine Methylgruppe darstellt und worin |
| Δ | eine 4(5)-Doppelbindung symbolisiert, falls |
| Z | zwei Wasserstoffatome oder eine Oxogruppe bedeutet oder eine 5(6)- oder im Falle der Estran-Derivate der allgemeinen Formel I auch eine 5(10)-Doppelbindung symbolisiert, falls Z eine Alkylendioxygruppe bedeutet, |

mit der Maßgabe, daß X ein Jodatom darstellt, falls Y zwei Wasserstoffatome, Z eine Oxogruppe V eine Ethylengruppe und $R_2$ eine Methylgruppe bedeutet.

Insbesondere betrifft die Erfindung Estran-Derivate der allgemeinen Formel I a

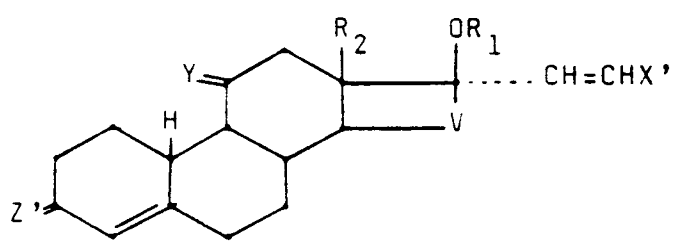

(Ia),

worin

X' ein Bromatom oder ein Iodatom darstellt Z' zwei Wasserstoffatome oder eine Oxogruppe und V, Y, $R_1$ und $R_2$ die im Anspruch 1 genannte Bedeutung besitzen,

mit der Maßgabe, daß X' ein Jodatom darstellt, falls Y zwei Wasserstoffatome, Z' eine Oxogruppe, V eine Ethylengruppe und $R_2$ eine Methylgruppe bedeutet.

Die Verbindungen der allgemeinen Formel I gemäß Patentanspruch 1 können als Substituenten $R_1$ ein Wasserstoffatom oder einen gegebenenfalls durch eine Oxygruppe unterbrochenen oder durch eine Oxogruppe substituierten Kohlenwasserstoffrest mit maximal 8 Kohlenstoffatomen tragen. Solche Reste sind beispielsweise gesättigte oder ungesättigte Kohlenwasserstoffreste wie Alkylreste (Methyl, Ethyl, Propyl, Isopropyl, tert.-Butyl etc.) Alkenylreste (2-Propenyl etc.) oder der Benzylrest, Acetal- oder Ketalreste

(Methoxymethyl, Ethoxymethyl, 2-Tetrahydrofuranyl, 2-Tetrahydropyranyl etc.), 1-Oxoalkylreste (Formyl, Acetyl, Propionyl, Butyryl, Trimethylacetyl, Hexanoyl etc.) oder der Benzoylrest.

Als Trialkylzinngruppe X der Verbindungen der allgemeinen Formel I gemäß Patentanspruch 1 kommt vorzugsweise die Tributylzinngruppe in Betracht.

Geeignete Alkylendioxygruppen Z der Verbindungen der Formel I gemäß Patentanspruch 1 sind beispielsweise die Ethylendioxygruppe, die 1,3-Propylendioxygruppe, die 2,2-Dimethylpropylendioxygruppe oder die 2,3-Butylendioxygruppe.

Die Verbindungen der allgemeinen Formel I gemäß Patentanspruch 1 sind, falls X ein Bromatom oder ein Jodatom darstellt und Z zwei Wasserstoffatome oder eine Oxogruppe bedeutet, pharmakologisch wirksame Substanzen, die ein Wirkungsprofil zeigen wie die entsprechenden 17α-Ethinylverbindungen. So haben beispielsweise die Verbindungen der allgemeinen Formel I a eine gestagene Wirksamkeit. So wird beispielsweise das 17β-Hydroxy-17α-(2-iodvinyl)-4-estran-3-on wesentlich stärker am Gestagenrezeptor gebunden als das Norethisteron.

Die Verbindungen der allgemeinen Formel I gemäß Patentanspruch 1 mit X in der Bedeutung einer Triphenylzinngruppe oder einer Trialkylzinngruppe und/oder mit Z in der Bedeutung einer Alkylendioxygruppe sind vorzugsweise Zwischenprodukte zur Herstellung der pharmakologisch wirksamen Substanzen.

Die Herstellung der erfindungsgemäßen Estran- und Androstan-Derivate kann beispielsweise nach einem Verfahren erfolgen,
dadurch gekennzeichnet
allgemeinen Formel II

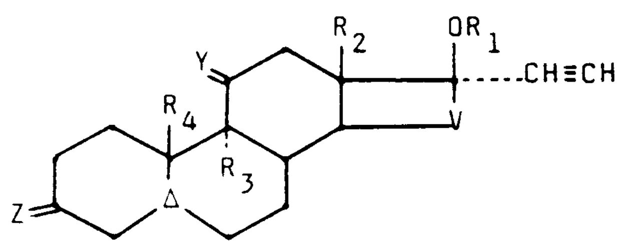

(II),

worin
Y, Z, V, Δ, $R_1$, $R_2$, $R_3$ und $R_4$ die im Anspruch 1 genannte Bedeutung besitzen, mit Triphenylzinnhydrid oder einem Trialkylzinnhydrid mit 1 bis 6 Kohlenstoffatomen je Alkylrest umsetzt und gewünschtenfalls den Organozinnrest gegen Brom oder Iod austauscht und eine vorhandene Ketalgruppe unter gleichzeitiger Isomerisierung der Doppelbindung spaltet.

Die Umsetzung der Verbindung der allgemeinen Formel II mit Triphenylzinnhydrid oder einem Trialkylzinnhydrid mit 1 bis 6 Kohlenstoffatomen je Alkylrest wird in einem inerten Lösungsmittel durchgeführt. Geeignete Lösungsmittel sind beispielsweise Ether wie Diethylether, Dioxan, Tetrahydrofuran, 1,2-Dimethoxyethan, Dimethylformamid, Hexamethylphosporsäuretriamid, N-Methylpyrrolidon, Acetonitril oder Isopropanol. Die Reaktion kann ohne Zusatz von Katalysatoren oder in Gegenwart von Radikalbildnern (wie zum Beispiel das α,α'-Azoisobutyronitril) als Katalysator durchgeführt werden. Führt man die Reaktion in Gegenwart von Radikalbildnern durch, ist es zweckmäßig, eine 3,3-Alkylendioxyverbindung oder ein in der 3-Position unsubstituiertes Steroid der allgemeinen Formel II als Ausgangsprodukt einzusetzen, da sonst die Gefahr besteht, daß die vorhandene $\Delta^4$-Doppelbindung hydriert wird. Wird die Reaktion in Gegenwart von Radiaklbildnern durchgeführt entstehen im allgemeinen die 17α-(E-2-Tributylstannylvinyl)-Verbindungen der allgemeinen Formel I gemäß Patentanspruch 1. Ohne Radikalbildner werden im allgemeinen die 17α-(Z-2-Tributylstannylvinyl)-Verbindungen gebildet.

Der sich gegebenenfalls anschließende Austausch der Triphenylzinngruppe oder Trialkylzinngruppe gegen ein Brom- oder Iodatom wird in inerten Lösungsmitteln in Gegenwart von Bromkationen oder Iodkationen liefernden Agentien durchgeführt. Eine geeignete Methode ist beispielsweise die Umsetzung der zinnorganischen Verbindungen in einem inerten Lösungsmittel (zum Beispiel einem Ether wie Dioxan, Tetrahydrofuran oder 1,2-Dimethoxyethan) mit N-Bromsuccinimid oder N-Iodsuccinimid. Andere Methoden sind beispielsweise die in den Publikationen J. Nucl. Med. 23, 1982, 431, Appl. Nucl. Radiochem. 1982, 197 oder Helv. Chim. Acta,66, (1983) 1018 beschriebenen Verfahren.

Die sich gewünschtenfalls anschließende Ketalspaltung wird in üblicher Weise mittels Säuren (Salzsäure, p-Toluolsulfonsäure, Oxalsäure, Ameisensäure etc.) in Gegenwart von hydroxygruppenhaltigen Lösungsmitteln (Wasser, niederen Alkoholen wie Methanol, Ethanol, Isopropanol) oder Lösungsmittelgemischen durchgeführt.

Die 17α-(2-Bromvinyl)- und 17α-(2-Iodvinyl)-Steroide der allgemeinen Formel I gemäß Patentanspruch 1 können Ausgangsprodukte für die Herstellung der entsprechenden 17α-(2-Radiohalogenvinyl)-Verbindungen sein. So lassen sich nach einer neuen radiochemischen Methode an 17α-(2-Iodvinyl)- bzw. 17α-(2-Bromvinyl)-Steroiden durch Austauschreaktion mit radioaktivem Natrium-[*I]-iodid in Aceton 17α-(2-[*I]-Iodvinyl-Verbindungen herstellen. Analog bilden sich aus 17α-Iod- und 17α-Bromvinyl-Verbindungen mit radioaktivem Natrium-[*Br]-bromid unter Zusatz von Kupfersulfat die entsprechenden 17α-(2-[*Br]-Bromvinyl-Steroide. Eine weitere, literaturbekannte Methode zur Herstellung von 17α-(2-[*I]-Iodvinyl)-Steroiden verläuft über die 17α-(2-Tributylstannylvinyl)-Verbindungen, die mit Radioiod, z.B. [$^{125}$I]-$I_2$, umgesetzt werden. (R.N. Hanson et al., J.Nucl. Med. 23, 341 (1982)).

Geeignete radioaktive Iodisotope sind beispielsweise das $^{124}$I-, $^{125}$I-, $^{126}$I- oder $^{131}$I-Isotop, geeignete radioaktive Bromisotope sind beispielsweise das $^{77}$Br- und $^{82}$Br-Isotop.

Die so hergestellten radioaktiven Verbindungen sind wertvolle Diagnostika. So eignen sich beispielsweise die Verbindungen der allgemeinen Formel I a infolge ihrer Affinität zum Gestagenrezeptor in der Medizin für diagnostische Zwecke. Sie können als Radiodiagnostika eingesetzt werden. In der Szintigraphie lassen sie sich zur Darstellung von Organen und Tumoren, die Rezeptoren enthalten, welche von den Vinylhalogen-Steroiden besetzt werden, verwenden. Bei den Organen handelt es sich bei der Frau z.B. um die Geschlechtsorgane, insbesondere Uterus und Milchdrüsen; beim Mann sind es hauptsächlich Prostata, akzessorische Geschlechtsorgane, Hypophyse und Brustdrüse. Weiterhin können die Radiohalogenvinyl-Steroide zur Auffindung von Tumoren und Metastasen in den aufgeführten Organgeweben eingesetzt werden. Von besonderem Interesse sind die 17α-[$^{125}$I]-Iodvinyl-Verbindungen, da sie sich für die in-vitro Diagnostika eignen.

Die nachfolgenden Ausführungsbeispiele dienen zur näheren Erläuterung der Erfindung.

Beispiel 1

17β-Hydroxy-17α-(Z-2-tributylstannylvinyl)-4-estren-3-on

10,0 g 17α-Ethinyl-17β-hydroxy-4-estren-3-on werden in 200 ml Tetrahydrofuran mit 30 ml Tributylzinnhydrid bei 70° C gerührt. Nach 4 Tagen wird das Lösungsmittel abdestilliert und der Rückstand an Kieselgel mit 0-10 % Essigester/Hexan chromatographiert. Es werden 7,5 g 17β-Hydroxy-17α-(Z-2-tributylstannylvinyl)-4-estren-3-on erhalten. Schmelzpunkt 114,2° C.

Beispiel 2

17β-Hydroxy-17α-(Z-2-iodvinyl)-4-estren-3-on

Zu 710 mg 17β-Hydroxy-17α-(2-tributylstannylvinyl)-4-estren-3-on in 14 ml Tetrahydrofuran gibt man bei Raumtemperatur 355 mg N-Iodsuccinimid. Nach 15 Minuten tropft man das Gemisch in Eis/Wasser. Man filtriert das ausgefallene Produkt ab, löst in Essigester, wäscht mehrmals mit Wasser und trocknet über Natriumsulfat. Nach Umkristallisieren des Rohproduktes aus Aceton/Hexan werden 520 mg 17β-Hydroxy-17α-(Z-2-iodvinyl)-4-estren-3-on erhalten. Schmp. 125.1°C (Zers.)

Beispiel 3

17α-(Z-2-Bromvinyl)-17β-hydroxy-4-estren-3-on

1.5 g 17β-Hydroxy-17α-(2-tributylstannylvinyl)-4-estren-3-on in 30 ml Tetrahydrofuran werden bei Raumtemperatur mit 530 mg N-Bromsuccinimid innerhalb 40 Minuten umgesetzt. Das Reaktionsgemisch wird in Eis/Wasser eingerührt und das ausgefallene Produkt wie im Beispiel 2 beschrieben aufgearbeitet. Ausbeute: 770 mg 17α-(Z-2-Bromvinyl)-17β-hydroxy-4-estren-3-on. Schmp. 124.0°C (Zers.)

Beispiel 4

17α-Ethinyl-3,3-ethylendioxy-5 und 5(10)-estren-17β-ol

10 g 17α-Ethinyl-17β-hydroxy-4-estren-3-on in 100 ml Methylenchlorid werden bei Raumtemperatur mit 100 ml Ethylenglykol, 30 ml o-Ameisensäuretrimethylester und 150 mg p-Toluolsulfonsäure gerührt. Nach 5

Stunden setzt man 2,5 ml Pyridin zu, engt im Vakuum die Lösung weitgehend ein und gibt den Rückstand in Eis/Wasser. Das ausgefallene Produkt wird abgesaugt, in Essigester gelöst, mit Wasser gewaschen und über Natriumsulfat getrocknet. Man erhält 11,6 g 17α-Ethinyl-3,3-ethylendioxy-5 und 5(10)-estren-17β-ol als Öl.

Beispiel 5

3.3-Ethylendioxy-17α-(E-2-tributylstannylvinyl)-5 und 5(10)-estren-17β-ol

2.0 g 17α-Ethinyl-3.3-ethylendioxy-5 und 5(10)-estren-17β-ol werden in 40 ml abs. Tetrahydrofuran mit 10 ml n-Tributylzinnhydrid und 400 mg α,α'-Azoisobutyronitril 1 Stunde bei 70°C gerührt. Das Reaktionsgemisch wird nach dem Abkühlen mit Essigester verdünnt, mit Wasser gewaschen und über Natriumsulfat getrocknet. Nach Säulenchromatographie an Kieselgel mit 0-10% Essigester/Hexan werden 1.8 g 3.3-Ethylendioxy-17α-(E-2-tributylstannylvinyl)-5 und 5(10)-estren-17β-ol als Öl isoliert.

Beispiel 6

3.3-Ethylendioxy-17α-(E-2-iodvinyl)-5- und 5(10)-estren-17β-ol

Zu 1.5 g 3.3-Ethylendioxy-17α-(E-2-tributylstannylvinyl)-5 und 5(10)-estren-17β-ol in 30 ml abs. Tetrahydrofuran gibt man bei Raumtemperatur 770 mg N-Iodsuccinimid. Nach 1 Stunde verdünnt man mit Essigester und trocknet über Natriumsulfat. Nach Umkristallisieren aus Ether/Hexan werden 800 mg 3.3-Ethylendioxy-17α-(E-2-iodvinyl)-5 und 5(10)-estren-17β-ol erhalten. Schmp. 172.2°C (Zers.)

Beispiel 7

17α-(E-2-Bromvinyl)-3.3-ethylendioxy-5 und 5(10)-estren-17β-ol

1.5 g 3.3-Ethylendioxy-17α-(E-2-tributylstannylvinyl)-5 und 5(10)-estren-17ß-ol werden analog Beispiel 6 mit 540 mg N-Bromsuccinimid umgesetzt und aufgearbeitet. Es werden 700 mg 17α-(E-2-Bromvinyl-3.3-ethylendioxy-5 und 5(10)-estren-17ß-ol vom Schmp. 154.3°C (Zers.) erhalten.

Beispiel 8

17β-Hydroxy-17α-(E-2-iodvinyl)-4-estren-3-on

1.3 g 3.3-Ethylendioxy-17α-(E-2-iodvinyl)-5 und 5(10)-estren-17β-ol werden in einem Gemisch aus 35 ml Methanol und 2.5 ml Wasser mit 1.2 g Oxalsäure 15 Minuten am Rückfluß gerührt. Das Reaktionsgemisch wird in Eis/Wasser gegeben. Das ausgefallene Produkt wird abgesaugt, in Essigester gelöst, mit Wasser gewaschen und über Natriumsulfat getrocknet. Nach Umkristallisieren aus Aceton/Hexan werden 1.0 g 17β-Hydroxy-17α-(E-2-iodvinyl)-4-estren-3-on erhalten. Schmelzp. 130°C (Zers.)

Beispiel 9

17α-(E-2-Bromvinyl)-17β-hydroxy-4-estren-3-on

1.3 g 17α-(E-2-Bromvinyl)-3.3-ethylendioxy-5 und 5(10)-estren-17β-ol werden analog Beispiel 8 zum 17α-(E-2-Bromvinyl)-17β-hydroxy-4-estren-3-on umgesetzt. Ausbeute: 830 mg, Schmp. 132°C (Zers.)

Beispiel 10

17β-Hydroxy-18-methyl-17α-(Z-2-tributylstannylvinyl)-4-estren-3-on

3 g 17α-Ethinyl-17β-hydroxy-18-methyl-4-estren-3-on werden analog Beispiel 1 mit Tributylzinnhydrid umgesetzt. Es werden nach Chromatographieren des Rohproduktes an Kieselgel mit 0-10% Essigester/Hexan 1.9 g 17β-Hydroxy-18-methyl-17α-(Z-2-tributylstannylvinyl)-4-estren-3-on erhalten. Schmelzp. 120-121 ° C.

Beispiel 11

17$\beta$-Hydroxy-17$\alpha$-(Z-2-iodvinyl)-18-methyl-4-estren-3-on

1.0 g 17$\beta$-Hydroxy-18-methyl-17$\alpha$-(Z-2-tributylstannylvinyl)-4-estren-3-on werden in Analogie zum Beispiel 2 mit N-Iodsuccinimid umgesetzt. Es werden 580 mg 17$\beta$-Hydroxy-17$\alpha$-(Z-2-iodvinyl)-18-methyl-4-estren-3-on erhalten.

Beispiel 12

17$\alpha$-(Z-2-Bromvinyl)-17$\beta$-hydroxy-18-methyl-4-estren-3-on

500 mg 17$\beta$-Hydroxy-18-methyl-17$\alpha$-(Z-2-tributylstannylvinyl)-4-estren-3-on werden in Analogie zu Beispiel 3 mit N-Bromsuccinimid zu 17$\alpha$-(Z-2-Bromvinyl)-17$\beta$-hydroxy-18-methyl-4-estren-3-on umgesetzt. Ausbeute: 310 mg.

Beispiel 13

3.3-(2.2-Dimethyltrimethylendioxy)-18-methyl-17$\alpha$-(E-2-tributylstannylvinyl)-5 und 5(10),15-estradien-17$\beta$-ol

1.3 g 3.3-(2.2-Dimethyltrimethylendioxy-18-methyl-5 und 5(10), 15-estradien-17$\beta$-ol (US-Patent 4081537) werden analog Beispiel 5 zu 3.3-(2.2-Dimethyltrimethylendioxy)-18-methyl-17$\alpha$-(E-2-tributylstannylvinyl)-5 und 5(10),15-estradien-17$\beta$-ol umgesetzt. Ausbeute: 890 mg als Öl.

Beispiel 14

3.3-(2.2-Dimethyltrimethylendioxy)-17$\alpha$-(E-2-iodvinyl)-18-methyl-5 und 5(10),15-estradien-17$\beta$-ol

730 mg 3.3-(2.2-Dimethyltrimethylendioxy)-18-methyl-17$\alpha$-(E-2-tributylstannylvinyl)-5 und 5(10),15-estradien-17$\beta$-ol werden analog Beispiel 6 mit N-Iodsuccinimid umgesetzt. Es werden 430 mg 3.3-(2.2-Dimethyltrimethylendioxy)-17$\alpha$-(E-2-iodvinyl)-18-methyl-5 und 5(10)-estradien-17$\beta$-ol als Öl isoliert.

Beispiel 15

17$\alpha$-(E-2-Bromvinyl)-3.3-(2.2-dimethyltrimethylendioxy)-18-methyl-5 und 5(10),15-estradien-17$\beta$-ol

520 mg 3.3-(2.2-Dimethyltrimethylendioxy)-18-methyl-17$\alpha$-(E-2-tributylstannylvinyl)-5 und 5(10),15-estradien-17$\beta$-ol werden analog Beispiel 7 zu 17$\alpha$-(E-2-Bromvinyl)-3.3-(2.2-dimethyltrimethylendioxy)-18-methyl-5 und 5(10), 15-estradien-17$\beta$-ol umgesetzt. Ausbeute: 270 mg als Öl.

Beispiel 16

17$\beta$-Hydroxy-17$\alpha$-(E-2-iodvinyl)-18-methyl-4,15-estradien-3-on

360 mg 3.3-(2.2-Dimethyltrimethylendioxo)-17$\alpha$-(E-2-iodvinyl)-18-methyl-5 und 5(10),15-estradien-17$\beta$-ol werden analog Beispiel 8 zu 17$\beta$-Hydroxy-17$\alpha$-(E-2-iodvinyl)-18-methyl-4,15-estradien-3-on umgesetzt. Ausbeute 210 mg.

Beispiel 17

17$\alpha$-(E-2-Bromvinyl)-17$\beta$-hydroxy-18-methyl-4,15-estradien-3-on

200 mg 17$\alpha$-(E-2-Bromvinyl)-3.3-(2.2-dimethyl-trimethylendioxy)-18-methyl-5 und 5(10),15-estradien-17$\beta$-ol werden analog Beispiel 8 zu 130 mg 17$\alpha$-(E-2-Bromvinyl)-17$\beta$-hydroxy-18-methyl-4,15-estradien-3-on umgesetzt.

Beispiel 18

18-Methyl-11-methylen-17α-(Z-2-tributylstannylvinyl)-4-estren-17β-ol

2.5 g 17α-Ethinyl-18-methyl-11-methylen-4-estren-17β-ol (DT 2361120 (1974)) werden analog Beispiel 1 mit Tributylzinnhydrid umgesetzt. Nach Chromatographieren an Kieselgel mit 0-5% Essigester/Hexan werden 1.7 g 18-Methyl-11-methylen-17α-(Z-2-tributylstannylvinyl)-4-estren17β-ol als Öl erhalten.

Beispiel 15

17α-(Z-2-Iodvinyl)-18-methyl-11-methylen-4-estren-17β-ol

760 mg 18-Methyl-11-methylen-17α-(2-tributylstannylvinyl)-4-estren-17β-ol werden analog Beispiel 2 mit N-Iodsuccinimid umgesetzt. Es werden 320 mg 17α-(Z-2-Bromvinyl)-18-methyl-11 -methylen-4-estren-17β-ol.

Beispiel 20

17α-(Z-2-Bromvinyl)-18-methyl-11-methylen-4-estren-17β-ol

630 mg 18-Methyl-11-methylen-17α-(Z-2-tributylstannylvinyl)-4-estren-17β-ol werden analog Beispiel 3 mit N-Bromsuccinimid umgesetzt. Man erhält 390 mg 17α-(Z-2-Bromvinyl)-18-methyl-11-methylen-4-estren-17β-ol.

Beispiels 21

17α-Ethinyl-3,3-(2,2-dimethyltrimethylendioxy)-18-methyl-11-methylen-5-estren-17β-ol

3,2 g 17α-Ethinyl-17β-hydroxy-18-methyl-11-methylen-4-estren-3-on (DT 2361120 (1974)) in 40 ml Methylenchlorid werden bei Raumtemperatur mit 3 g 2,2-Dimethyl-1,3-propandiol, 4 ml o-Ameisensäuretriethylester und 400 mg p-Toluolsulfonsäure gerührt. Nach 3 Stunden verdünnt man die Lösung mit Methylenchlorid und wäscht nacheinander mit Natriumhydrogencarbonat-Lösung und Wasser. Das Rohprodukt wird an Kieselgel mit 0-15 % Aceton/Hexan chromatographiert. Es werden 2,6 g 17α-Ethinyl-3,3-(2,2-dimethyltrimethylendioxy)-18-methyl-11-methylen-5-estren-17β-ol erhalten. Schmelzpunkt 196,2° C.

Beispiel 22

3.3-(2.2-Dimethyltrimethylendioxy)-18-methyl-11-methylen-17α-(E-2-Tributylstannylvinyl)-5-estren-17β-ol

1.4 g 17α-Ethinyl-3.3-(2.2-dimethyltrimethylendioxy)-18-methyl-11-methylen-5-estren-17β-ol werden analog Beispiel 5 zu 3.3-(2.2-Dimethyltrimethylendioxy)-18-methyl-11-methylen-17α-(2-tri-n-butylstannylvinyl)-5-estren-17β-ol umgesetzt. Nach Chromatographieren des Rohproduktes an Kieselgel mit 0-10% Essigester/Hexan werden 1.1 g 3.3-(2.2-Dimethyl-trimethylendioxy)-18-methyl-11-methylen-17α-(E-2-tributylstannylvinyl)-5-estren-17β-ol als Öl erhalten.

Beispiel 23

3.3-(2.2-Dimethyltrimethylendioxy)-17α-(E-2-iodvinyl)-18-methyl-11-methylen-5-estren-17β-ol

460 mg 3.3-(2.2-Dimethyltrimethylendioxy)-18-methyl-11-methylen-17α-(E-2-tributylstannylvinyl)-5-estren-17β-ol werden analog Beispiel 6 mit N-Iodsuccinimid umgesetzt. Es werden 280 mg 3.3-(2.2 Dimethytrimethylendioxy)-17α-(E-2-iodvinyl)-18-methyl-11-methylen-5-estren-17β-ol als Öl erhalten.

Beispiel 24

17α-(E-2-Bromvinyl)-3,3-(2,3-dimethyltrimethylendioxy)-18-methyl-11-methylen-5-estren-17β-ol

510 mg 3.3-(2.2-Dimethyltrimethylendioxy)-18-methyl-11-methylen-17α-(E-2-tributylstannylvinyl)-5-estren-17β-ol werden analog Beispiel 7 mit N-Bromsuccinimid umgesetzt. Es werden 300 mg 17α-(E-2-Bromvinyl)-3.3-(2.2-trimethylendioxy)-18-methyl-11-methylen-5-estren-17β-ol als Schaum isoliert.

Beispiel 25

17β-Hydroxy-17α-(E-2-iodvinyl)-18-methyl-11-methylen-4-estren-3-on

210 mg 3,3-(2,2-Dimethyltrimethylendioxy)-17α-(E-2-iodvinyl)-18-methyl-11-methylen-5-estren-17β-ol werden analog Beispiel 8 umgesetzt. Es werden 130 mg 17β-Hydroxy-17α-(E-2-iodvinyl)-18-methyl-11-methylen-4-estren-3-on erhalten.

Beispiel 26

17α-(E-2-Bromvinyl)-17β-hydroxy-18-methyl-11-methylen-4-estren-3-on

17α-(E-2-Bromvinyl)-17β-hydroxy-18-methyl-11-methylen-4-estren-3-on

250 mg 17α-(-E-2-Bromvinyl)-3.3-(2.2-trimethylendioxy)-18-methyl-11-methylen-5-estren-17β-ol werden analog Beispiel 8 zu 17α-(E-2-Bromvinyl)-17β-hydroxy-18-methyl-11-methylen-4-estren-3-on umgesetzt. Ausbeute 140 mg.

Beispiel 27

17β-Hydroxy-17α-(Z-2-tributylstannylvinyl)-4-androsten-3-on

4.5 g 17α-Ethinyl-17β-hydroxy-4-androstan-3-on werden analog Beispiel 1 mit Tributylzinnhydrid umgesetzt. Nach Chromatographieren des Rohproduktes an Kieselgel mit 0-20% Essigester/Hexan werden 2.6 g 17β-Hydroxy-17α-(Z-2-tributylstannylvinyl)-4-androstan-3-on als Öl erhalten.

Beispiel 28

17β-Hydroxy-17α-(Z-2-iodvinyl)-4-androsten-3-on

1.4 g 17β-Hydroxy-17α-(Z-2-tributylstannylvinyl)-4-androsten-3-on werden analog Beispiel 2 mit N-Iodsuccinimid umgesetzt, wobei 710 mg 17β-Hydroxy-17α-(Z-2-iodvinyl)-4-androsten-3-on erhalten werden.

Beispiel 29

3,3-Ethylendioxy-17β-tetrahydropyranyloxy-17α-(E-2-tributylstannylvinyl)-5-estren

5,6 g 17α-Ethinyl-3,3-ethylendioxy-17β-tetrahydropyranyloxy-5-estren (DA-ES 1242697 (1967)) werden analog Beispiel 5 umgesetzt. Nach Chromatographieren des Rohproduktes an Kieselgel mit 0-10 % Essigester/Hexan erhält man 3,9 g 3,3-Ethylendioxy-17β-tetrahydropyranyloxy-17α-(E-2-tributylstannylvinyl)-5-estren als Öl.

Beispiel 30

3,3-Ethylendioxy-17α-(E-2-idovinyl)-17β-tetrahydropyranyloxy-5-estren

3,1 g 3,3-Ethylendioxy-17β-tetrahydropyranyloxy-17α-(E-2-tributylstannylvinyl)-5-estren werden analog Beispiel 2 mit N-Iodsuccinimid zu 3,3-Ethylendioxy-(17α-(E-2-iodvinyl)-17β-tetrahydropyranyloxy-5-estren umgesetzt. Es werden 1,9 g als Öl erhalten.

Beispiel 31

17β-Hydroxy-17α-(E-2-iodvinyl)-4-estren-3-on

Aus 1.6 g 3.3-Ethylendioxy-17α-(E-2-iodvinyl)-17β-tetrahydropyranyloxy-5-estren werden analog Beispiel 8 710 mg 17β-Hydroxy-17α-(E-2-iodvinyl)-4-estren-3-on erhalten. Schmp. 124.6°C.

Beispiel 32

17β-Acetoxy-3.3-ethylendioxy-17α-(E-2-tributylstannylvinyl)-5 und 5(10)-estren

6.3 g 17β-Acetoxy-3.3-ethylendioxy-5 und 5(10)-estren [Tetrahedron 20, 2295 (1964)] werden analog Beispiel 5 mit Tributylzinnhydrid umgesetzt. Es werden nach Chromatographieren des Rohproduktes an Kieselgel mit 0-15% Essigester/Hexan 4.7 g 17β-Acetoxy-3.3-ethylendioxy-17α-(E-2-tributylstannylvinyl)-5 und 5(10)-estren als Öl erhalten.

Beispiel 33

17β-Acetoxy-3,3-ethylendioxy-17α-(E-2-iodvinyl)-5- und 5(10)-estren

3,6 g 17β-Acetoxy-3,3-ethylendioxy-17α-(E-2-tributylstannylvinyl)- und 5(10)-estren werden in Analogie zu Beispiel 6 mit N-Iodsuccinimid umgesetzt. Es werden 2,3 g 17β-Acetoxy-3,3-ethylendioxy-17α-(E-2-iodvinyl)-5 und 5(10)-estren als Schaum erhalten.

Beispiel 34

17β-Acetoxy-17α-(E-2-iodvinyl)-4-estren-3-on

1,9 g 17β-Acetoxy-3,3-ethylendioxy-17α-(E-2-iodvinyl)-5 und 5(10)-estren werden analog Beispiel 8 umgesetzt. Man erhält 890 mg 17β-Acetoxy-17α-(E-2-iodvinyl)-4-estren-3-on.

Beispiel 35

17aβ-Hydroxy-17aα-(Z-2-tributylstannylvinyl)-D-homo-4-estren-3-on

7.3 g 17aα-Ethinyl-17aβ-hydroxy-D-homo-4-estren-3-on (US-Pat. 3850911 (1974] werden analog Beispiel 1 umgesetzt. Nach Chromatographieren an Kieselgel mit 0-20% Essigester/Hexan erhält man 4.2 g 17aβ-Hydroxy-17aα-(Z-2-tributylstannylvinyl)-D-homo-4-estren-3-on als Schaum.

Beispiel 36

17αβ-Hydroxy-17aα-(Z-2-iodvinyl)-D-homo-4-estren-3-on

800 mg 17aβ-Hydroxy-17aα-(Z-2-tributylstannylvinyl)-D-homo-4-estren-3-on werden analog Beispiel 2 mit N-Iodsuccinimid umgesetzt. Man erhält 210 mg 17αβ-Hydroxy-17aα-(Z-2-iodvinyl)-D-homo-4-estren-3-on.

Beispiel 37

3.3-(2.2-Dimethyltrimethylendioxy)-18-methyl-15α,16α-methylen-17α-(E-2-tributylstannylvinyl)-5 und 5(10)-estren-17β-ol

5.8 g 17α-Ethinyl-3.3-(2.2-dimethyltrimethylendioxy)-18-methyl-15α,16α-methylen-5 und 5(10)-estren-17β-ol (US-Pat. 3994937) werden analog Beispiel 5 umgesetzt. Nach Chromatographieren des Rohproduktes an Kieselgel mit 0-15% Essigester/Hexan werden 3.9 g 3.3-(2.2-Dimethyltrimethylendioxy-18-methyl-15α,16α-methylen-17α(E2-tributylstannylvinyl)-5 und 5(10)-estren-17β-ol als Öl erhalten.

Beispiel 38

3,3-(2,2-Dimethyltrimethylendioxy)-17α-(E-2-iodvinyl)-18-methyl-15α,16α-methylen-5 und 5(10)-estren-17β-ol

1,5 g 3,3-(2,2-Dimethyltrimethylendioxy)-18-methyl-15α,16α-methylen-17α-(E-2-tributylstannylvinyl)-5 und 5(10)-estren-17β-ol werden analog Beispiel 6 mit N-Iodsuccinimid umgesetzt. Man erhält 830 mg 3,3-(2,2-Dimethyltrimethylendioxy)-17α-(E-2-iodvinyl)-18-methyl-15α,16α-methylen-5 und 5(10)-estren-17β-ol als schaumiges Produkt.

Beispiel 39

17α-(E-2-Bromvinyl)-3.3-(2.2-dimethyltrimethylendioxy)-18-methyl-15α,16α-methylen-5 und 5(10)-estren-17β-ol

850 mg 3.3-(2.2-Dimethyltrimethylendioxy)-18-methyl-15α,16α-methylen-17α-(E-2-tributylstannylvinyl)-5 und 5(10)-estren-17β-ol werden analog Beispiel 7 mit N-Bromsuccinimid zu 17α-(E-2-Bromvinyl)-3.3-(2.2-dimethyl-trimethylendioxy)-18-methyl-15α,16α-methylen-5 und 5(10)-estren-17β-ol umgesetzt. Ausbeute: 410 mg als Schaum.

Beispiel 40

17β-Hydroxy-17α-(E-2-iodvinyl)-18-methyl-15α,16α-methylen-4-estren-3-on

Aus 650 mg 3.3(2.2-Dimethyltrimethylendioxy)-17α-(E-2-iodvinyl)-18-methyl-15α,16α-methylen-5 und 5-(10)-estren-17β-ol werden analog Beispiel 8 390 mg 17β-Hydroxy-17α(E2-iodvinyl)-18-methyl-15α,16α-methylen-4-estren-3-on als schaumiges Produkt erhalten.

Beispiel 41

17α-(E-2-Bromvinyl)-17β-hydroxy-18-methyl-15α,16α-methylen-4-estren-3-on

Aus 300 mg 17α-(E-2-Bromvinyl)-3.3(2.2-dimethyltrimethylendioxy)-18-methyl-15α,16α-methylen-5 und 5(10)-estren-17β-ol werden analog Beispiel 8 130 mg 17α-(E-2-Bromvinyl)-17β-hydroxy-18-methyl-15α,16α-methylen-4-estren-3-on als Schaum erhalten.

Beispiel 42

17β-Hydroxy-18-methyl-15β,16β-methylen-17α-(Z-2-tributylstannylvinyl)-4-estren-3-on

4,3 g 17α-Ethinyl-17β-hydroxy-18-methyl-15β,16β-methylen-4-estren-3-on (DT 1643050) werden analog Beispiel 1 mit Tributylzinnhydrid umgesetzt. Nach Chromatographie des Rohproduktes an Kieselgel mit 0-15 % Essigester/Hexan werden 2,3 g 17β-Hydroxy-18-methyl-15β,16β-methylen-17α-(Z-2-tributylstannylvinyl)-4-estren-3-on als Öl erhalten.

Beispiel 43

17β-Hydroxy-17α-(Z-2-iodvinyl)-18-methyl-15β,16β-methylen-4-estren-3-on

560 mg 17β-Hydroxy-18-methyl-15β,16β-methylen-17α-(Z-2-tributylstannylvinyl)-4-estren-3-on werden analog Beispiel 2 mit N-Iodsuccinimid umgesetzt. Es werden 280 mg 17β-Hydroxy-17α-(Z-2-iodvinyl)-18-methyl-15β,16β-methylen-4-estren-3-on als Schaum erhalten.

Beispiel 44

17α-(Z-2-Bromvinyl)-17β-hydroxy-18-methyl-15β,16β-methylen-4-estren-3-on

Aus 380 mg 17β-Hydroxy-18-methyl-15β,16β-methylen-17α-(Z-2-tributylstannylvinyl)-4-estren-3-on werden analog Beispiel 3 110 mg 17α-(Z-2-Bromvinyl)-17β-hydroxy-18-methyl-15β,16β-methylen-4-estren-3-on als Schaum isoliert.

Beispiel 45

17β-Hydroxy-17α-(Z-2-[$^{131}$I]-iodvinyl)-4-estren-3-on

5 µg (0,11 µMol) 17β-Hydroxy-17α-(Z-2-iodvinyl)-4-estren-3-on werden in 0,25 ml Aceton (über $P_2O_5$ dest.) gelöst und unter Schutzgas in Gegenwart von 370 MBq Natrium-[$^{131}$I]-iodid höchster spezifischer Aktivität eine Stunde unter Rückfluß erhitzt.

Nach Abkühlen bis auf Eisbadtemperatur setzt man 0,5 ml Methanol, 0,1 ml Wasser und 1 µg Natriumiodid zu. Zum Entfernen der Ionen zieht man die Lösung durch einen Mischbett-Ionenaustauscher

oder trennt im DC-System Dioxan/Ammoniak/Wasser. Man erhält 4 µg 17β-Hydroxy-17α-(Z-2-[$^{131}$I]-iodvinyl)-4-estren-3-on bei einer ca. 50 %igen radiochemischen Ausbeute mit einer spezifischen Aktivität von 185 MBq/4 µg (19,5 GBq/µMol). Die chemische und radiochemische Identität der Verbindung wird durch Co-Chromatographie auf DC-Platten und analoge Retentionszeiten in der HPLC bestätigt. Für die diagnostische bzw. therapeutische Anwendung engt man die Lösung unter Vakuum ein, löst in Ethanol/Propylenglykol und schließt eine Steril-Filtration an. Auf analoge Weise erhält man die $^{124}$I-, $^{125}$I- und $^{132}$I-Verbindung.

Beispiel 46

5 µg (0,013 µMol) 17α-(Z-2-Bromvinyl)-17β-hydroxy-4-estren-3-on werden in 0,25 ml Diethylenglycoldiethylether (trocken) gelöst und unter Schutzgas in Gegenwart von 370 MBq Natrium-[$^{125}$I]-iodid (trägerfrei) und 1 µg Kupfersulfat 3 Stunden auf 120° C erhitzt.

Nach Abkühlen auf Eisbadtemperatur setzt man 0,25 ml Methanol, 0,1 ml Wasser und 1 g Natriumiodid zu. Zum Entfernen der Ionen zieht man die Lösung durch einen Mischbett-Ionenaustauscher. Man erhält 2 µg 17β-Hydroxy-17α-(Z-2-[$^{125}$I]-iodvinyl)- 4-estren-3-on mit einer spezifischen Aktivität, die der des eingesetzten Natrium-[$^{125}$I]-iodid entspricht. Sie liegt somit in der Größenordnung von 80,29 GBq/µMol. Das erhaltene, mit $^{125}$I trägerfrei markierte 17β-Hydroxy-17α-(Z-2-iodvinyl)- 4-estren-3-on wird vom Ausgangsprodukt durch Chromatographie an Silicagel auf einer Niederdrucksäule im System Hexan/Aceton (Aceton 10→ 30 %) abgetrennt.

Nach Einengen, Aufnehmen in Ethanol/Propylenglykol, erhält man ein für diagnostische Zwecke geeignetes Produkt.

Beispiel 47

Wie für Beispiel 45 beschrieben, behandelt man 50 µg (0,13 µMol) 17β-Hydroxy-17α-(Z-2-bromvinyl)-4-estren-3-on mit Natrium- [$^{82}$Br]-bromid (trägerfrei). Man erhält nach analoger Aufarbeitung spezifisch hoch markiertes 17β-Hydroxy-17α-(Z-2-bromvinyl)-4-estren-3-on.

Entsprechend wird die $^{77}$Br, $^{80m}$Br, $^{80}$Br, markierte Verbindung erhalten.

Beispiel 48

Zu 500 µg 17β-Hydroxy-17α-(Z-2-tributylstannylvinyl)-4-estren-3-on in 250 µl Methyläthylketon werden bei Zimmertemperatur 50 µl Na-$^{125}$I, enthaltend 185 MBq $^{125}$I,als wässrig neutrale Lösung zugegeben. Man läßt 5 min bei Zimmertemperatur stehen, trennt die entstandenen Stoffe auf einer analytischen HPTLC-Platte und eluiert die radioaktive Zone. Das Eluat wird über eine halbpräparative HPLC-Säule mit Radioaktivitätsmonitor aufgetrennt. Man erhält 1 mCi reines 17β-Hydroxy-17α-(Z-2-$^{125}$iodvinyl)-4-estren- 3-on in methanolisch wässriger Lösung. Die radioaktive Markierung ist trägerfrei. Die spezifische Aktivität entspricht der des eingesetzten radioaktiven Iods.

Analog wie für $^{125}$Iod beschrieben, erhält man die Verbindung auch mit $^{123}$Iod, $^{131}$Iod und $^{132}$Iod markiert, ebenso mit $^{77}$Brom, $^{80m}$Brom und $^{82}$Brom.

Beispiel 49

Zu 500 µg 17β-Hydroxy-17α-(Z-2-tributylstannylvinyl)-18-methyl-4-estren-3-on in 250 µl Methyläthylketon werden bei Zimmertemperatur 50 µl Na-$^{125}$I, enthaltend 185 MBq $^{125}$I, als wässrig neutrale Lösung zugegeben. Man läßt 5 min bei Zimmertemperatur stehen, trennt die entstandenen Stoffe auf einer analytischen HPTLC-Platte und eluiert die radioaktive Zone. Das Eluat wird über eine halbpräparative HPLC-Säule nlage mit Radioaktivitätsmonitor aufgetrennt. Man erhält 1 mCi reines 17β-Hydroxy-17α-(Z-2-$^{125}$iodvinyl)- 18-methyl-4-estren-3-on in methanolisch wässriger Lösung. Die radioaktive Markierung ist trägerfrei. Die spezifische Aktivität entspricht der des eingesetzten radioaktiven Iods.

Analog wie für $^{125}$Iod beschrieben, erhält man die Verbindung auch mit $^{123}$Iod, $^{131}$Iod und $^{132}$Iod markiert, ebenso mit $^{77}$Brom, $^{80m}$Brom und $^{82}$Brom.

Beispiel 50

3,3-Ethylendioxy-17α-(E-2-tributylstannylvinyl)-5(10),9(11)-17β-ol

5,0 g 17α-Ethinyl-3,3-ethylendioxy-5(10),9(11)-estradien-17β-ol werden analog Beispiel 5 umgesetzt. Es werden 5,9 g 3,3-Ethylendioxy-17α-(E-2-tributylstannylvinyl)-5(10),9(11)-estradien-17β-ol als Öl erhalten.

Beispiel 51

3,3-Ethylendioxy-17α-(E-2-iodvinyl)-5(10),9(11)-estradien-17β-ol

2,0 g 3,3-Ethylendioxy-17α-(E-2-tributylstannylvinyl)-5(10),9(11)-estradien-17β-ol werden analog Beispiel 6 umgesetzt. Es werden 1,4 g 3,3-Ethylendioxy-17α-(E-2-iodvinyl)-5(10),9(11)-estradien-17β-ol als Schaum erhalten.

Beispiel 52

17α-(E-2-Bromvinyl)-3,3-ethylendioxy-5(10),9(11)-estradien-17β-ol

2,0 g 3,3-Ethylendioxy-17α-(E-2-tributylstannylvinyl)-5(10),9(11)-estradien-17β-ol werden analog Beispiel 7 umgesetzt. Es werden 1,8 g 17α-(E-2-Bromvinyl)-3,3-ethylendioxy-5(10),9(11)-estradien-17β-ol als Öl isoliert.

Beispiel 53

17β-Hydroxy-17α-(E-2-iodvinyl)-4,9-estradien-3-on

1,3 g 3,3-Ethylendioxy-17α-(E-2-iodvinyl)-5(10),9(11)-estradien-17β-ol werden analog Beispiel 8 umgesetzt. Es werden 650 mg 17β-Hydroxy-17α-(E-2-iodvinyl)-4,9-estradien-3-on als Schaum erhalten.

Beispiel 54

17α-(E-2-Bromvinyl)-17β-hydroxy-4,9-estradien-3-on.

1,0 g 17α-(E-2-Bromvinyl)-3,3-ethylendioxy-5(10),9(11)-estradien-17β-ol werden analog Beispiel 8 umgesetzt. Es werden 600 mg 17α-(E-2-Bromvinyl)-17β-hydroxy-4,9-estradien-3-on erhalten. Schmelzp. 112° C (Zersetzung).

Beispiel 55

3,3-Ethylendioxy-17α-(Z-2-tributylstannylvinyl)-5(10),9(11)-estradien-17β-ol

7,0 g 17α-Ethinyl-3,3-ethylendioxy-5(10),9(11)-estradien-17β-ol werden in 30 ml Hexamethylphosphorsäuretriamid und 30 ml Tributylzinnhydrid 6 Stunden bei 70° C gerührt. Man verdünnt mit Essigester, wäscht mehrmals mit Wasser und trocknet über \atriumsulfat. Das Rohprodukt wird mit Toluol-Essigester an Kieselgel chromatographiert. Es werden 4,2 g 3,3-Ethylendioxy-17α-(Z-2-tributylstannylvinyl)-5(10),9(11)-estradien-17β-ol als Öl isoliert.

Beispiel 56

3,3-Ethylendioxy-17α-(Z-2-iodvinyl)-5(10),9(11)-estradien-17β-ol

1,5 g 3,3-Ethylendioxy-17α-(Z-2-tributylstannylvinyl)-5(10),9(11)-estradien-17β-ol werden analog Beispiel 2 umgesetzt. Es werden 650 mg 3,3-Ethylendioxy-17α-(Z-2-iodvinyl)-5(10),9(11)-estradien-17β-ol als Schaum isoliert.

Beispiel 57

17α-(Z-2-Bromvinyl)-3,3-ethylendioxy-5(10),9(11)-estradien-17β-ol

1,2 g 3,3-Ethylendioxy-17α-(Z-2-tributylstannylvinyl)-5(10),9(11)-estradien-17β-ol werden analog Beispiel 3 umgesetzt. Es werden 480 mg 17α-(Z-2-Bromvinyl)-3,3-ethylendioxy-5(10),9(11)-estradien-17β-ol als

Schaum erhalten.

Beispiel 58

17β-Hydroxy-17α-(Z-2-iodvinyl)-4,9-estradien-3-on

500 mg 3,3-Ethylendioxy-17α-(Z-2-iodvinyl)-5(10),9(11)-estradien-17β-ol werden analog Beispiel 8 umgesetzt. Es werden 280 mg 17β-Hydroxy-17α-(Z-2-iodvinyl)-4,9-estradien-3-on erhalten. Schmelzp. 98° C (Zersetzung).

Beispiel 59

17α(Z-2-Bromvinyl)-17β-hydroxy-4,9-estradien-3-on

350 mg 17α-(Z-2-Bromvinyl)-3,3-ethylendioxy-5(10),9(11)-estradien-17β-ol werden analog Beispiel 8 umgesetzt. Es werden 120 mg 17α-(Z-2-Bromvinyl)-17β-hydroxy-4,9-estradien-3-on als Schaum erhalten.

**Patentansprüche**

**1.** Estran- und Androstan-Derivate der allgemeinen Formel I

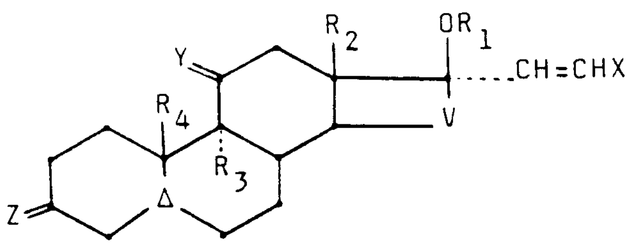

(I),

worin

| | |
|---|---|
| X | ein Bromatom, ein Iodatom, eine Triphenylzinngruppe oder eine Trialkylzinngruppe mit 1 bis 6 Kohlenstoffatomen je Alkylrest, |
| Y | zwei Wasserstoffatome oder eine Methylengruppe, |
| Z | zwei Wasserstoffatome, eine Oxogruppe oder eine Alkylendioxygruppe mit 2 bis 6 Kohlenstoffatomen und |
| V | eine Ethylengruppe, eine Vinylengruppe, eine 1,3-Propylengruppe oder eine Cyclopropylengruppe darstellen, worin |
| $R_1$ | ein Wasserstoffatom oder einen gegebenenfalls durch eine Oxygruppe unterbrochenen oder durch eine Oxogruppe substituierten Kohlenwasserstoffrest mit maximal 8 Kohlenstoffatomen und |
| $R_2$ | eine Methyl- oder Ethylgruppe bedeuten, worin |
| $R_3$ und $R_4$ | eine Kohlenstoff-Kohlenstoff-Bindung darstellt oder worin $R_3$ ein Wasserstoffatom bedeutet und |
| $R_4$ | ein Wasserstoffatom oder eine Methylgruppe darstellt und worin |
| Δ | eine 4(5)-Doppelbindung symbolisiert, falls |
| Z | zwei Wasserstoffatome oder eine Oxogruppe bedeutet oder eine 5(6)- oder im Falle der Estran-Derivate der allgemeinen Formel I auch eine 5(10)-Doppelbindung symbolisiert, falls Z eine Alkylendioxygruppe bedeutet, mit der Maßgabe, daß X ein Jodatom darstellt, falls Y zwei Wasserstoffatome, Z eine Oxogruppe V eine Ethylengruppe und $R_2$ eine Methylgruppe bedeutet. |

**2.** Estran-Derivate der allgemeinen Formel I a

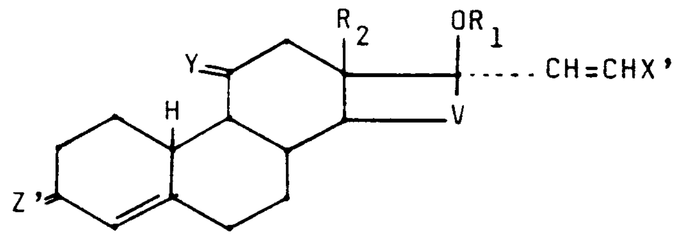

(Ia),

worin

X' ein Bromatom oder ein Iodatom darstellt Z' zwei Wasserstoffatome oder eine Oxogruppe und V, Y, $R_1$ und $R_2$ die im Anspruch 1 genannte Bedeutung besitzen, mit der Maßgabe, daß X' ein Jodatom darstellt, falls Y zwei Wasserstoffatome, Z' eine Oxogruppe, V eine Ethylengruppe und $R_2$ eine Methylgruppe bedeutet.

**3.** 17β-Hydroxy-17α-(2-iodvinyl)-4-estren-3-on.

**4.** 17α-(2-Bromvinyl)-17β-hydroxy-18-methyl-4-estren-3-on.

**5.** 17β-Hydroxy-17α-(2-iodvinyl)-18-methyl-4-estren-3-on.

**6.** 17α-(2-Bromvinyl)-17β-hydroxy-18-methyl-4,15-estradien-3-on.

**7.** 17β-Hydroxy-17α-(2-iodvinyl)-18-methyl-4,15-estradien-3-on.

**8.** 17α-(2-Bromvinyl)-18-methyl-4-estren-17β-ol.

**9.** 17α-(2-Iodvinyl)-18-methyl-4-estren-17β-ol.

**10.** 17α-(2-Bromvinyl)-17β-hydroxy-18-methyl-11-methylen-4-estren-3-on.

**11.** 17β-Hydroxy-17α-(2-iodvinyl)-18-methyl-11-methylen-4-estren-3-on.

**12.** 17β-Hydroxy-17α-(2-iodvinyl)-4-androsten-3-on.

**13.** 17β-Acetoxy-17α-iodvinyl-4-estren-3-on.

**14.** 17aα-Hydroxy-17aα-iodvinyl-D-homo-4-estren-3-on.

**15.** 17α-(2-Bromvinyl)-17β-hydroxy-18-methyl-15α,16α-methylen-4-estren-3-on.

**16.** 17β-Hydroxy-17α-iodvinyl-18-methyl-15α,16α-methylen-4-estren-3-on.

**17.** 17α-(2-Bromvinyl)-17β-hydroxy-18-methyl-15β,16β-methylen-4-estren-3-on.

**18.** 17β-Hydroxy-17α-iodvinyl-18-methyl-15β,16β-methylen-4-estren-3-on.

**19.** Estran-Derivate gemäß Anspruch 2 bis 11 und 13 bis 18, dadurch gekennzeichnet, daß sie ein radioaktives Bromisotop oder ein radioaktives Iodisotop enthalten.

**20.** Diagnostisches Mittel gekennzeichnet durch einen Gehalt an einem Estran-Derivat gemäß Patentanspruch 19.

**21.** Verfahren zur Herstellung von Estran- und Androstan-Derivaten der allgemeinen Formel I gemäß Patentanspruch 1, dadurch gekennzeichnet, daß man eine Verbindung der allgemeinen Formel II

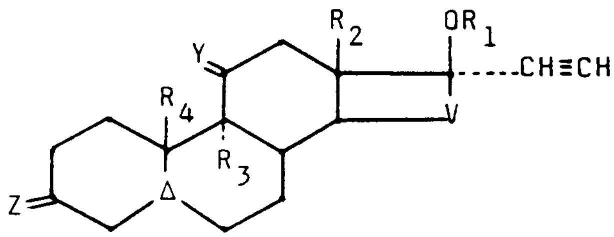

(II),

worin
Y, Z, V, Δ, $R_1$, $R_2$, $R_3$ und $R_4$ die im Anspruch 1 genannte Bedeutung besitzen, mit Triphenylzinnhydrid oder einem Trialkylzinnhydrid mit 1 bis 6 Kohlenstoffatomen je Alkylrest umsetzt und gewünschtenfalls den Organozinnrest gegen Brom oder Iod austauscht und eine vorhandene Ketalgruppe unter gleichzeitiger Isomerisierung der Doppelbindung spaltet.

**22.** 3,3-Ethylendioxy-17α-(2-tributylstannyl)-5(10),9(11)-estradien-17β-ol.

**23.** 3,3-Ethylendioxy-17α-(2-iodvinyl)-5(10),9(11)-estradien-17β-ol.

**24.** 17α-(2-bromvinyl)-3,3-ethylendioxy-5(10),9(11)-estradien-17β-ol.

**25.** 17β-Hydroxy-17α-(2-iodvinyl)-4,9-estradien-3-on.

**26.** 17α-(2-Bromvinyl)-17β-hydroxy-4,9-estradien-3-on.

**Claims**

**1.** Oestrane and androstane derivatives of the general formula I

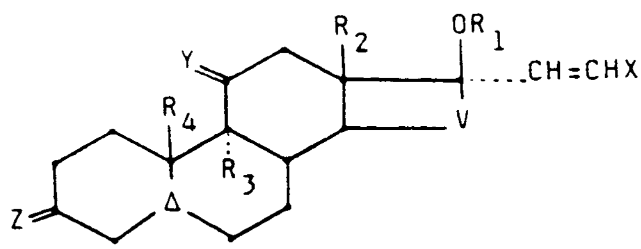

(I)

wherein
- X represents a bromine atom, an iodine atom, a triphenyltin group, or a trialkyltin group having from 1 to 6 carbon atoms per alkyl radical,
- Y represents two hydrogen atoms or a methylene group,
- Z represents two hydrogen atoms, an oxo group or an alkylenedioxy group having from 2 to 6 carbon atoms, and
- V represents an ethylene group, a vinylene group, a 1,3-propylene group or a cyclopropylene group, wherein
- $R_1$ represents a hydrogen atom, or a hydrocarbon radical having a maximum of 8 carbon atoms that is optionally interrupted by an oxy group or substituted by an oxo group, and
- $R_2$ represents a methyl or ethyl group, wherein
- $R_3$ and $R_4$ represent a carbon-carbon bond, or wherein
- $R_3$ represents a hydrogen atom and
- $R_4$ represents a hydrogen atom or a methyl group, and wherein
- Δ represents a 4(5)-double bond when Z represents two hydrogen atoms or an oxo group, or represents a 5(6)-double bond or, in the case of the oestrane derivatives of the general formula I, a 5(10)-double bond when Z represents an alkylenedioxy group,

with the proviso that X represents an iodine atom when Y represents two hydrogen atoms, Z represents an oxo group, V represents an ethylene group and $R_2$ represents a methyl group.

2. Oestrane derivatives of the general formula Ia

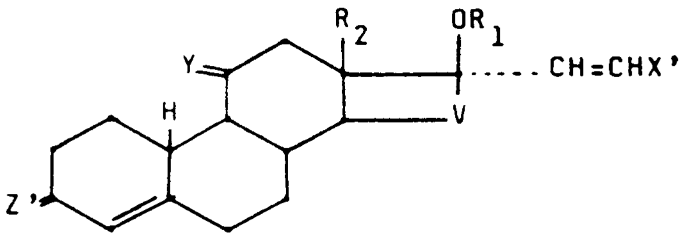

(Ia)

wherein
X' represents a bromine atom or an iodine atom, Z' represents two hydrogen atoms or an oxo group, and V, Y, $R_1$ and $R_2$ have the meanings given in claim 1, with the proviso that X' represents an iodine atom when Y represents two hydrogen atoms, Z' represents an oxo group, V represents an ethylene group and $R_2$ represents a methyl group.

3. 17β-hydroxy-17α-(2-iodovinyl)-4-oestren-3-one.

4. 17α-(2-bromovinyl)-17β-hydroxy-18-methyl-4-oestren-3-one.

5. 17β-hydroxy-17α-(2-iodovinyl)-18-methyl-4-oestren-3-one.

6. 17α-(2-bromovinyl)-17β-hydroxy-18-methyl-4,15-oestradien-3-one.

7. 17β-hydroxy-17α-(2-iodovinyl)-18-methyl-4,15-oestradien-3-one.

8. 17α-(2-bromovinyl)-18-methyl-4-oestren-17β-ol.

9. 17α-(2-iodovinyl)-18-methyl-4-oestren-17β-ol.

10. 17α-(2-bromovinyl)-17β-hydroxy-18-methyl-11-methylene-4-oestren-3-one.

11. 17β-hydroxy-17α-(2-iodovinyl)-18-methyl-11-methylene-4-oestren-3-one.

12. 17β-hydroxy-17α-(2-iodovinyl)-4-androsten-3-one.

13. 17β-acetoxy-17α-iodovinyl-4-oestren-3-one.

14. 17aα-hydroxy-17aα-iodovinyl-D-homo-4-oestren-3-one.

15. 17α-(2-bromovinyl)-17β-hydroxy-18-methyl-15α,16α-methylene-4-oestren-3-one.

16. 17β-hydroxy-17α-iodovinyl-18-methyl-15α,16α-methylene-4-oestren-3-one.

17. 17α-(2-bromovinyl)-17β-hydroxy-18-methyl-15β,16β-methylene-4-oestren-3-one.

18. 17β-hydroxy-17α-iodovinyl-18-methyl-15β,16β-methylene-4-oestren-3-one.

19. Oestrane derivatives according to claims 2 to 11 and 13 to 18, characterised in that they contain a radioactive bromine isotope or a radioactive iodine isotope.

20. Diagnostic agent, characterised in that it contains an oestrane derivative according to patent claim 19.

**21.** Process for the preparation of oestrane and androstane derivatives of the general formula I according to patent claim 1, characterised in that a compound of the general formula II

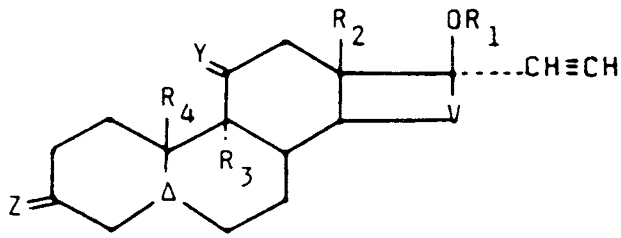

(II),

wherein Y, Z, V, Δ, $R_1$, $R_2$, $R_3$ and $R_4$ have the meanings given in claim 1, is reacted with triphenyltin hydride or with a trialkyltin hydride having from 1 to 6 carbon atoms per alkyl radical and, if desired, the organotin radical is replaced by bromine or iodine and a ketal group that is present is removed with simultaneous isomerisation of the double bond.

**22.** 3,3-ethylenedioxy-17α-(2-tributylstannyl)-5(10),9(11)-oestradien-17β-ol.

**23.** 3,3-ethylenedioxy-17α-(2-iodovinyl)-5(10),9(11)-oestradien-17β-ol.

**24.** 17α-(2-bromovinyl)-3,3-ethylenedioxy-5(10),9(11)-oestradien-17β-ol.

**25.** 17β-hydroxy-17α-(2-iodovinyl)-4,9-oestradien-3-one.

**26.** 17α-(2-bromovinyl)-17β-hydroxy-4,9-oestradien-3-one.

**Revendications**

**1.** Dérivés de l'estrane et de l'androstane répondant à la formule générale I :

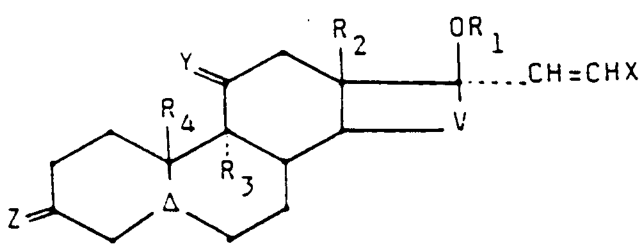

(I),

dans laquelle

| | |
|---|---|
| X | représente un atome de brome, un atome d'iode, un radical triphényl-étain ou un radical trialkyl-étain contenant de 1 à 6 atomes de carbone dans chacun de ses alkyles, |
| Y | représente deux atomes d'hydrogène ou un radical méthylène, |
| Z | représente deux atomes d'hydrogène, un radical oxo ou un radical alkylène-dioxy contenant de 2 à 6 atomes de carbone, |
| V | représente un radical éthylène, un radical vinylène, un radical propylène-1,3 ou un radical cyclopropylène, |
| $R_1$ | représente un atome d'hydrogène ou un radical hydrocarboné contenant au plus 8 atomes de carbone, radical qui est éventuellement interrompu par un radical oxy ou qui porte éventuellement un radical oxo comme substituant, |
| $R_2$ | représente un radical méthyle ou éthyle, |
| $R_3$ et $R_4$ | représentent ensemble une liaison carbone-carbone ou $R_3$ représente un atome d'hydrogène et $R_4$ un atome d'hydrogène ou un radical méthyle et |
| Δ | représente une double liaison 4(5) lorsque Z désigne deux atomes d'hydrogène ou un radical oxo, ou représente une double liaison 5(6) ou encore, dans le cas des dérivés |

de l'estrane de formule générale I, une double liaison 5(10), lorsque Z représente un radical alkylènedioxy,

avec la condition que X représente un atome d'iode lorsque Y représente deux atomes d'hydrogène, que Z représente un radical oxo, que V représente un radical éthylène et que $R_2$ représente un radical méthyle.

**2.** Dérivés de l'estrane répondant à la formule générale Ia :

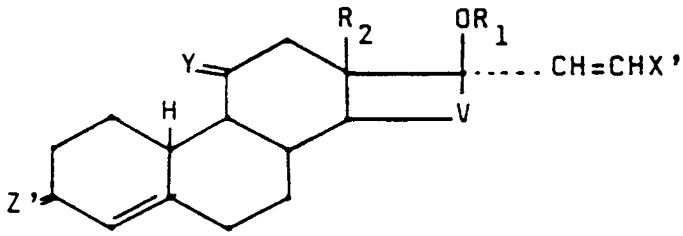

(Ia),

dans laquelle X' représente un atome de brome ou d'iode, Z' représente deux atomes d'hydrogène ou un radical oxo, et V, Y, $R_1$ et $R_2$ ont les significations qui leur ont été données à la revendication 1, avec la condition que X' représente un atome d'iode lorsque Y représente deux atomes d'hydrogène, Z' un radical oxo, V un radical éthylène et $R_2$ un radical méthyle.

**3.** Hydroxy-17β (iodo-2 vinyl)-17α estrène-4 one-3.

**4.** (Bromo-2 vinyl)-17α hydroxy-17β méthyl-18 estrène-4 one-3.

**5.** Hydroxy-17β (iodo-2 vinyl)-17α méthyl-18 estrène-4 one-3.

**6.** (Bromo-2 vinyl)-17α hydroxy-17β méthyl-18 estradiène-4,15 one-3.

**7.** Hydroxy-17β (iodo-2 vinyl)-17α méthyl-18 estradiène-4,5 one-3.

**8.** (Bromo-2 vinyl)-17α méthyl-18 estrène-4 ol-17β.

**9.** (Iodo-2 vinyl)-17α méthyl-18 estrène-4 ol-17β.

**10.** (Bromo-2 vinyl)-17α hydroxy-17β méthyl-18 méthylène-11 estrène-4 one-3.

**11.** Hydroxy-17β (iodo-2 vinyl)-17α méthyl-18 méthylène-11 estrène-4 one-3.

**12.** Hydroxy-17β (iodo-2 vinyl)-17α androstène-4 one-3.

**13.** Acétoxy-17β (iodo vinyl)-17α estrène-4 one-3.

**14.** Hydroxy-17aβ (iodo vinyl)-17aα D-homo-estrène-4 one-3.

**15.** (Bromo-2 vinyl)-17α hydroxy-17β méthyl-18 méthylène-15α,16α estrène-4-one-3.

**16.** Hydroxy-17β (iodo vinyl)-17α méthyl-18 méthylène-15α,16α estrène-4 one-3.

**17.** (Bromo-2 vinyl)-17α hydroxy-17β méthyl-18 méthylène-15β,16β estrène-4 one-3.

**18.** Hydroxy-17β (iodo vinyl)-17α méthyl-18 méthylène-15β,16β estrène-4 one-3.

**19.** Dérivés de l'estrane selon l'une quelconque des revendications 2 à 11 et 13 à 18, caractérisés en ce qu'ils contiennent un isotope radioactif du brome ou un isotrope radioactif de l'iode.

**20.** Agent de diagnostic caractérisé en ce qu'il contient un dérivé de l'estrane selon la revendication 19.

**21.** Procédé pour préparer des dérivés de l'estrane et de l'androstane répondant à la formule générale I selon la revendication 1, procédé caractérisé en ce qu'on fait réagir un composé répondant à la formule générale II :

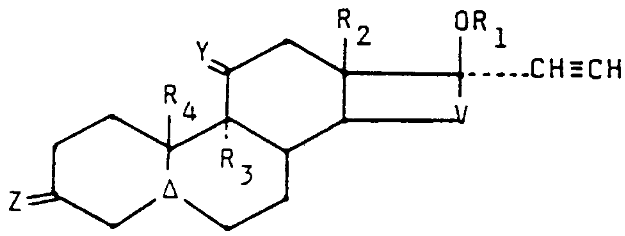

(II),

dans laquelle Y, Z, V, Δ, $R_1$, $R_2$, $R_3$ et $R_4$ ont les significations qui leur ont été données à la revendication 1, avec l'hydrure de triphényl-étain ou un hydrure de trialkylétain dont chacun des alkyles contient de 1 à 6 atomes de carbone, et, si on le désire, on échange le radical organostannique contre du brome ou de l'iode et on coupe un éventuel radical- acétal en même temps qu'on isomérise la double liaison.

**22.** Ethylène-dioxy-3,3 (tributylstannyl-2 vinyl)-17$\alpha$ estradiène-5(10),9(11) ol-17$\beta$.

**23.** Ethylène-dioxy-3,3 (iodo-2 vinyl)-17$\alpha$ estradiène-5(10),9(11) ol-17$\beta$.

**24.** (Bromo-2 vinyl)-17$\alpha$ éthylène-dioxy-3,3 estradiène-5(10), 9(11) ol-17$\beta$.

**25.** Hydroxy-17$\beta$ (iodo-2 vinyl)-17$\alpha$ estradiène-4,9 one-3.

**26.** (Bromo-2 vinyl)-17$\alpha$ hydroxy-17$\beta$ estradiène-4,9 one-3.